(19) Europäisches Patentamt
European Patent Office
Office européen des brevets

(11) **EP 2 000 169 A2**

(12) **EUROPEAN PATENT APPLICATION**
published in accordance with Art. 153(4) EPC

(43) Date of publication:
**10.12.2008 Bulletin 2008/50**

(51) Int Cl.:
***A61M 25/00*** *(2006.01)*

(21) Application number: **07739437.7**

(22) Date of filing: **23.03.2007**

(86) International application number:
**PCT/JP2007/055996**

(87) International publication number:
**WO 2007/111244 (04.10.2007 Gazette 2007/40)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **29.03.2006 JP 2006091491**

(71) Applicant: **Kaneka Corporation**
**Kita-ku**
**Osaka-shi, Osaka 530-8288 (JP)**

(72) Inventors:
• **MICHISHITA, Ichiro**
**Yokohama-shi**
**Kanagawa 247-0007 (JP)**
• **NISHIDE, Takuji**
**Settsu-shi**
**Osaka 566-0072 (JP)**

(74) Representative: **Gille Hrabal Struck Neidlein Prop Roos**
**Patentanwälte**
**Brucknerstrasse 20**
**40593 Düsseldorf (DE)**

(54) **CATHETER FOR BLOOD REMOVAL**

(57) A catheter for blood removal that is to be provided in the coronary sinus via the inferior vena cava to remove the blood in the coronary sinus from the body wherein: the catheter for blood removal has a straight-line back end shaft, a curved front end shaft connected in the front end side of the back end shaft, a hub connected in the back end side of the back end shaft and a blood removal lumen extending from the back end of the catheter for blood removal to the front end thereof; the front end shaft comprises a first straight-line part, a first curve part following the first straight-line part, a second curve part following the first curve part, a second straight-line part following the second curve part, a third curve part following the second straight-line part and a third curve part following the third straight-line part; at least one opening having an equivalent diameter of 1.8 mm or more is formed in the front end side of the apex of the second curve part; and the blood removal lumen has an equivalent diameter in the peripheral section of 1.8 mm or more throughout the full length of the blood removal lumen.

Fig. 1

**Description**

TECHNICAL FIELD

The present invention relates to a blood removal catheter used in medical applications, in particular to a blood removal catheter for removing blood containing a contrast medium locally administered to coronary artery from coronary sinus temporarily out of the body.

BACKGROUND ART

Angioplasty (PTA: Percutaneous Transluminal Angioplasty or PTCA: Percutaneous Transluminal Coronary Angioplasty) of improving blood flow in peripheral vessels by expanding a stenosis or occlusion site, for example with a balloon catheter, when stenosis or occlusion occurs in vascular systems including blood vessel or when blood vessel is obstructed by thrombus, has been practiced frequently in many medical institutions and thus practiced routinely in therapies of this kind of diseases.

Along with progress of DCA (Directional Coronary Atherectomy) and devices such as Rotor Brator, atherectomy therapy of dissecting atheroma by using a catheter is also practiced. In addition, devices for preservation of the enlarged stenosis or occlusion site such as stent are also used frequently. These therapies such as PTCA, atherectomy therapy and stent placement therapy are collectively called percutaneous coronary interventions (PCI). In recent advancement in surgeons' technical skill and device performance, more complicated lesions such as left main trunk (LMT) and chronic total occlusion (CTO) are now included in indications of PCI.

Contrast media are chemicals essential during coronary angiography (CAG) and PCI and have been used widely. On the other hand, contrast media are known to exhibit adverse reaction, causing renal dysfunction, dermatopathy, cardiovascular disorder, respiratory disorder, urinary disorder, and others. Thus, attempts to reduce the amount of the contrast medium administered are made, for example by using an injector.

However, in the case of complicated cases higher in difficulty such as LMT and CTO, the frequency of imaging increases, with accompanied increase in the amount of the contrast medium used. In addition, drug eluting stents (DES) of drastically preventing restenosis after stent placement operation were developed recently and gave many favorable therapeutic results, but placement of DES demands accurate estimation of the lesion such as the diameter and length of blood vessel and the position of DES relative to the lesion, which in turn demands use of a greater amount of contrast medium.

Recently, there are more reports on diabetes complication of the patients with PCI, and renal dysfunction is considered most significant among the adverse reactions of the contrast media. For prevention of such renal dysfunctions, which are called contrast medium-induced nephropathy collectively, administration of fluid replacement or a drug such as N-acetylcysteine and removal of the contrast medium by dialysis before and after PCI are practiced in particular for patients with renal failure.

In particular, dialysis was considered to the most effective means of removing the contrast media from blood, but there are some reports denying its effectiveness. As disclosed in Non-patent Document 1, there was no difference between the group of patients with chronic renal failure who are subjected to dialysis after use of a contrast medium (dialysis group) and the group of those without dialysis (non-dialysis group) in the frequency of contrast medium-induced nephropathy. The results indicate that the elongated period after administration of contrast medium to dialysis, which leads to circulation of contrast medium-containing blood in the body, is the reason for such renal dysfunction. Under the circumstances above, there exists a need for a treatment system alleviating the load on the kidney by the contrast medium during PCI, and the relevant methods are disclosed.

Patent Document 1 discloses a balloon catheter having an expandable balloon and a catheter main body including a catheter lumen extending from the proximal end to the distal region and a balloon lumen extending from the proximal end to the balloon, wherein multiple openings penetrating into the catheter lumen are formed at positions to the distal side relative to the balloon on the catheter main body.

A schematic cross-sectional view of the heart is shown in Figure 5. The catheter disclosed in Patent Document 1, which is placed in a coronary sinus ostium 26, is aimed, by administering a contrast medium into coronary artery and expanding the balloon simultaneously, at blocking the blood flow from the coronary sinus ostium 26 to a right atrium 25 and thus recovering the contrast medium-containing blood administered into coronary artery through the catheter lumen. However, the catheter disclosed in Patent Document 1 had the following problems.

First, it is difficult to expand the balloon in coronary sinus ostium 26 and to block the blood flow to the right atrium 25. Coronary artery extends through arteriolae and blood capillaries into venulae. Several venulae jointly form a great cardiac vein, a middle cardiac vein, and a small cardiac vein, which extend together with remaining venulae to coronary sinus and to right atrium. In this way, there are numerous veins connected to coronary sinus, and the inflow site spreads to a wide region close to the coronary sinus ostium 26. Thus if the balloon is expanded in coronary sinus, the blood flowing into the coronary sinus ostium 26 from venulae is sent to the right atrium 25 without being blocked, making it difficult to feed the blood into the catheter lumen.

In addition, coronary sinus wall is very thin, and thus, expansion of the balloon may cause damage or perforation of the wall. If the damage or perforation occurs, the blood spills into the region between heart and pericardiopleural membrane, possibly causing severe diseases such as cardiac tamponade.

On the other hand, it is significantly difficult to place a balloon accurately at a position covering coronary sinus ostium 26 reliably and also to fix the balloon there under heat beat. Therefore, in the case of the balloon catheter disclosed in Patent Document 1, it is difficult to block blood flow from coronary sinus to right atrium 25 and feed the blood into the catheter lumen, which in turn leads to decreased recovery rate of the contrast medium administered into coronary artery.

Patent Document 1: Japanese Unexamined Patent Publication No. 7-303701
Nonpatent Literature 1: Coronary Intervention, Vol. 2, No. 4, 2003, 78-83

Disclosure of the Invention

Problems to be Solved by the Invention

Under the circumstances above, an object of the present invention is to provide a blood removal catheter that allows efficient removal of the flowing from coronary artery via coronary vein into coronary sinus out of the body and operation similar to that of the catheters traditionally used in PCI.

Means to Solve the Problems

After intensive studies to solve the problems above, the inventors have found that it was possible to overcome the problems above by using the following catheter, and thus the invention was achieved.

It is a blood removal catheter for removing the blood in coronary sinus out of the body that is placed in coronary sinus through inferior vena cava, the blood removal catheter including: a straight proximal shaft; a curved distal shaft located to the distal side of the proximal shaft and connected to the straight proximal shaft; a hub connected to the proximal side of the proximal shaft; and a blood removal lumen extending in the blood removal catheter from the proximal end to the distal end, wherein the distal shaft includes a first straight region constituting the connection part to the proximal shaft; a first curved region connected to the distal side of the first straight region; a second curved region connected to the distal side of the first curved region and bending in the direction opposite to that of the first curved region; a second straight region connected to the distal side of the second curved region; a third curved region connected to the distal side of the second straight region and bending in the direction opposite to that of the second curved region; and a third straight region connected to the distal side of the third curved region and constituting the most distal region of the blood removal catheter, wherein the first and third curved regions form part of circular arcs; the second curved region forms part of an ellipse having a minor axis and a major axis; part of the ellipse has a starting point at one end of the minor axis and a terminal point at the other end of the minor axis; at least one opening having an equivalent diameter of 1.8 mm or more is formed at a position to the distal side from the vertex in the second curved region; and the equivalent diameter of the cross section of the blood removal lumen in the circumference direction is 1.8 mm or more over the entire length of the blood removal lumen.

The present invention also relates to the blood removal catheter, wherein the length L1 of the first straight region is 10 mm or more and 100 mm or less.

The present invention also relates to the blood removal catheter, wherein the first curved region is circular arc inside; the curvature radius R1 of the circular arc is 60 mm or more and 70 mm or less; and the circular arc angle θ1 is 30° or more and 50° or less.

The present invention also relates to the blood removal catheter, wherein the second curved region is partially elliptic inside; the ellipse has a major axis diameter A1 of 15 mm or more and 21 mm or less and a minor axis diameter B1 of 11 mm or more and 17 mm or less; and part of the ellipse extends from a starting point of one end of the minor axis to a terminal point at the other end of the minor axis.

The present invention also relates to the blood removal catheter, wherein the length L2 of the second straight region is 5 mm or more and 15 mm or less.

The present invention also relates to the blood removal catheter, wherein the third curved region is circular arc inside; the curvature radius R2 of the circular arc is 20 mm or more and 30 mm or less; and the circular arc angle θ2 is 30° or more and 50° or less.

The present invention also relates to the blood removal catheter, wherein the length L3 of the third straight region is 5 mm or more and 20 mm or less.

The present invention also relates to the blood removal catheter, wherein the distal shaft is made only of a resin material.

The present invention also relates to the blood removal catheter, wherein the resin contains an X ray-transmission-prohibiting substance as it is blended.

The present invention also relates to the blood removal catheter, wherein the proximal shaft is a composite tube in combination of a metal material and a resin material.

The present invention also relates to the blood removal catheter, wherein the metal material is a metal wire and the metal wire has a braid structure.

The present invention also relates to the blood removal catheter, wherein the metal material is a metal wire and the metal wire has a coil structure.

The present invention also relates to the blood removal catheter, further comprising a core wire detachably placed in the blood removal lumen.

The present invention also relates to the blood removal catheter, wherein the core wire has a connector at the proximal end and the connector is placed detachably from the hub.

The present invention also relates to the blood removal catheter, wherein the distal end of the core wire is located to the distal side of the opening.

Advantageous Effects of the Invention

According to the present invention, it is possible to remove blood containing a contrast medium administered into coronary artery out of the body efficiently through a blood removal catheter. The contrast medium contained in the blood removed can be eliminated by blood purification for example by dialysis or adsorption, and thus, it is possible to prevent renal dysfunction such as contrast medium-induced nephropathy effectively. In addition, the impression of using the blood removal catheter according to the present invention is not different from that of using catheters that have been traditionally used in PCI, and thus, it is possible to remove blood out of the body without excessive elongation of the operational period. Further, the blood removal catheter according to the present invention is extremely resistant to damaging to coronary sinus wall, when inserted into coronary sinus, and thus, can be used safely.

BRIEF DESCRIPTION OF THE DRAWINGS

Figure 1 is a schematic view illustrating an embodiment of the present invention.
Figure 2 is a regional schematic view illustrating an embodiment of the distal region according to the present invention.
Figure 3 is a regional schematic view illustrating an embodiment of the proximal region according to the present invention.
Figure 4 is a schematic view illustrating an embodiment of the core wire.
Figure 5 is a cross-sectional view illustrating a typical heart.

EXPLANATION OF REFERENCES

1 Distal shaft
2 Proximal shaft
3 Hub
4 Strain relief
5 Connector
6 Core wire
7 Opening
8 Blood removal lumen
9 First straight region
10 First curved region
11 Second curved region
12 Second straight region
13 Third curved region
14 Third straight region
15 Length of first straight region
16 Curvature radius of the circular arc inside first curved region

17 Arc angle of the circle of first curved region

18 Major axis of the ellipse of second curved region

19 Minor axis of the ellipse of second curved region

20 Length of second straight region

21 Curvature radius of the circular arc inside third curved region

22 Arc angle of the circle inside third curved region

23 Length of third straight region

24 Inferior vena cava

25 Right atrium

26 Coronary sinus ostium

27 Blood removal catheter

BEST MODE OF CARRYING OUT THE INVENTION

Hereinafter, various embodiments of the blood removal catheter according to the present invention will be described in detail with reference to drawings.

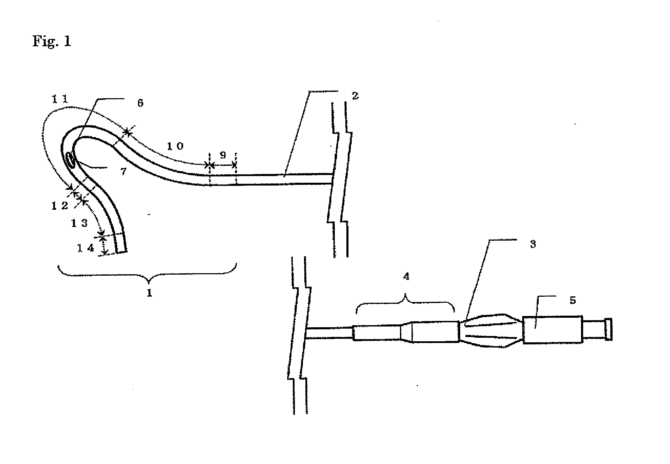

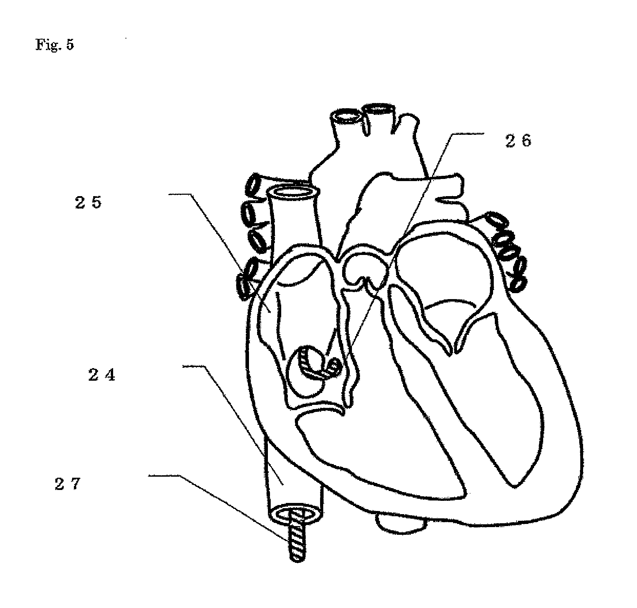

As shown in Figure 5, the catheter according to the present invention is a blood removal catheter 27 that is to be placed in coronary sinus via inferior vena cava 24 to remove the blood in the coronary sinus from the body. As shown in Figures 1 to 3, the blood removal catheter is characterized in that: it has a straight proximal shaft 2, a curved distal shaft 1 connected to the distal side of the proximal shaft 2, a hub 3 connected to the proximal side of the proximal shaft 2, and a blood removal lumen 8 extending from the proximal end to the distal end of the blood removal catheter 27; the distal shaft 1 has a first straight region 9 constituting the connection part to the proximal shaft 2, a first curved region 10 connected to the distal side of the first straight region 9, a second curved region 11 connected to the distal side of the first curved region 10 and bending in the direction opposite to that of the first curved region 10, a second straight region 12 connected to the distal side of the second curved region 11, a third curved region 13 connected to the distal side of the second straight region 12 and bending in the direction opposite to that of the second curved region 11, and a third straight region 14 connected to the distal side of the third curved region 13 and constituting the most distal region of the blood removal catheter 27; the first curved region 10 and the third curved region 13 correspond to part of circular arcs; the second curved region 11 is part of an ellipse having a minor axis 19 and a major axis 18; the part of the ellipse extends from a starting point at an end of the minor axis 19 to a terminal point at the other end of the minor axis 19; at least one opening 7 having an equivalent diameter of 1.8 mm or more is formed on the distal side from the vertex of the second curved region 11; and the blood removal lumen 8 has an equivalent diameter of 1.8 mm or more throughout the full length of the blood removal lumen 8.

The amount of the blood removed when blood is removed through a catheter can be predicted fluid-mechanically. When a fluid like blood flows through a tube such as a blood removal catheter, the Reynolds number Re, i.e., a dimensionless quantity indicating the flow rate in the tube, is defined by Formula 1. In the Formula, $S_m$ represents the sectional area of the blood removal lumen in the circumference direction; $U$ represents the blood flow rate in the blood removal lumen; $L_m$ represents the peripheral length in the cross section of the blood removal lumen in the circumference direction; $D_m$ represents the equivalent diameter of the cross section of the blood removal lumen in the circumference direction; $\rho$ represents the density of blood; and $\mu$ represents the viscosity of blood.

$$\text{(Formula 1)} \quad Re = (D_m \times U \times \rho)/\mu = [(4 \times S_m)/L_m] \times (\rho/\mu)$$

It is known that a flow is laminar when the Reynolds number is less than 2,100 and turbulent when it is more than 4,000. If the shape of the cross section of the blood removal lumen in the circumference direction is assumed to be a circle having a diameter of 3 mm, the Reynolds number is usually in the order of dozens Therefore, the blood flow in the blood removal lumen may be considered to be laminar flow.

The Hagen Poiseuille Formula shown by Formula 2 is applicable to tubular laminar flow. In the Formula, $L$ represents the length of the blood removal lumen; $\Delta P$ represents the absolute value of the pressure difference during blood removal (hereinafter, referred to as blood removal pressure); and $Q$ represents the amount of the blood removed. The blood removal pressure in the present invention is the pressure difference $P1-P0$ where the pressure at the blood removal rate of 0 mL/min is designated as $P0$ mmHg and the pressure at the blood removal rate of $Q1$ mL/min ($Q1>0$), as $P1$ mmHg.

$$\text{(Formula 2)} \quad Q=[\pi \times t\,(Dm/2)^4) \times \Delta\,P]/\,(8 \times \mu \times L)$$

When the contrast medium-containing blood administered into coronary artery is removed from coronary sinus and recirculated after purification, for example by adsorption for removal of the contrast medium, the blood removal rate is preferably higher, for more efficient removal of the contrast medium by blood purification. The blood removal rate is preferably at least 25 mL/min or more, more preferably 50 mL/min or more, and still more preferably 80 mL/min or more. The blood removal rate can be raised for example by a method of increasing the blood removal pressure by means of pump or syringe or a method of increasing the equivalent diameter of the blood removal lumen. Increase in blood removal rate is accompanied by decrease of P1 value, which leads to negative blood removal pressure. A blood removal pressure of lower than -200 mmHg, which may lead to clogging of blood vessel, is extremely dangerous and thus, the blood removal pressure is preferably -200 mmHg or higher. The blood removal pressure is more preferably -150 mmHg or higher, still more preferably -100 mmHg or higher, for prevention of flattening of the blood vessel.

The equivalent diameter Dm of the blood removal lumen is preferably 1.80 mm or more for assuring a blood removal rate of 80 mL/min or more under the condition of a blood removal pressure of -100 mmHg or higher. When Dm is less than 1.80 mm, it is difficult to obtain a blood removal rate of 80 mL/min or more consistently under the condition of a blood removal pressure of -100 mmHg or higher.

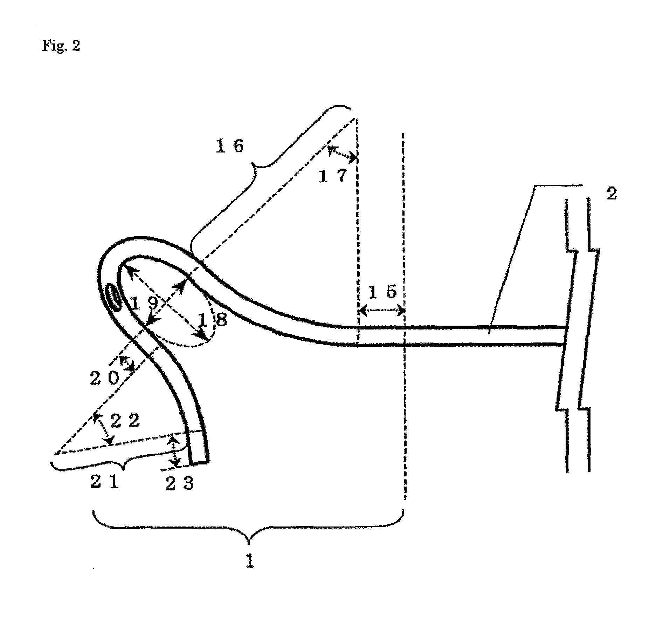

As shown in Figures 1 and 2, an opening 7 is preferably formed in the distal side from the vertex in the second curved region 11 of the blood removal catheter 27 according to the present invention. When a blood removal catheter 27 is inserted and placed for blood removal in the area surrounded by very thin wall such as coronary sinus, the distal region of the blood removal catheter 27 may be brought into tight contact with the surrounding wall under the influence of the blood removal pressure. Such tight contact prohibits the required blood removal rate and is also dangerous because of concern about the increased possibility of the damage and perforation of the surrounding wall. Because the blood removal pressure is raised to -100 mmHg or higher when the equivalent diameter of the cross section of the blood removal lumen 8 in the circumference direction is 1.80 mm or more, there is smaller possibility of the tight contact with the surrounding wall, and advantageously, presence of the opening 7 reduces the possibility further.

If the opening 7 is present in the proximal side of the most distal region of the blood removal catheter 27, blood removal is performed primarily through the opening 7. If the entire opening 7 is not located in coronary sinus, the blood removal is performed from right atrium 25, which leads to drastic drop in the efficiency of removing blood in coronary sinus. Therefore, it become possible to place at least part of the opening 7 in coronary sinus and remove the blood efficiently, by locating the opening 7 to the distal side from the vertex in the second curved region 11. It is also possible to achieve a blood removal rate of 80 mL/min or more consistently under the condition of a blood removal pressure of -100 mmHg or higher, by raising the equivalent diameter of the opening 7 to 1.8 mm or more.

Multiple openings 7 may be formed for more effective prevention of the tight contact with the surrounding wall of coronary sinus. Obviously, presence of the multiple openings 7 is effective for safe blood removal, unless all openings 7 are brought into tight contact with the surrounding wall.

On the other hand, the strength, kink resistance and flexibility of the distal shaft 1 vary according to the number of the openings 7 formed, the number of the openings 7 present on the same circumference of the distal shaft 1, and the shape of the openings 7. In the present invention, the opening 7 should only have an equivalent diameter of 1.80 mm or more, and the number of the openings 7, the number of the openings 7 present on the same circumference of the distal shaft 1, and the positional pattern when there are multiple openings 7 may be designed arbitrarily without any particular restriction.

The shape of the opening 7 does not restrict the advantageous effects of the present invention, and thus, the opening may have any shape, if the flexibility, strength, and other properties of the blood removal catheter 27 are suitable. Thus, the opening may be circular, elliptic (Figure 1) or rectangular in shape. When the shape is elliptic, as shown in Figure 1 the major axis direction may be in parallel with the axial direction or in the circumference direction of the blood removal catheter 27. Alternatively when it is rectangular, the long side direction may be in parallel with or perpendicular to the axial direction of the blood removal catheter 27. Moreover, all openings 7 may not be the same in shape, and circular, elliptic, rectangular and other shapes may be used in combination- The shape of the opening 7 is preferably circular or elliptic, for example considering the easiness in processing and deformation of the shape of the opening 7 when the blood removal catheter 27 is bent are considered.

The method of forming the opening 7 does not restrict the advantageous effects of the present invention. Laser processing such as by YAG laser, excimer laser or femtosecond laser or machining processing such as punching is used favorably.

The blood removal catheter 27 according to the present invention is placed in coronary sinus through inferior vena cava 24. The catheter may be introduced from any site if it is fed in the route through inferior vena cava 24, but is preferably introduced through a sheath introducer fixed to femoral vein. Normally, a device is introduced frequently through a sheath introducer fixed to the femoral artery during PCI. Therefore, it is possible to manipulate the catheter with a feeling similar to that of the PCI operation by using a sheath introducer fixed to femoral vein in introducing a blood removal catheter according to the present invention 27.

In addition, the blood removal catheter 27 according to the present invention preferably has a shape suitable for facile placement, because it is delivered though inferior vena cava 24 to coronary sinus.

When the length 19 of the first straight region 9 constituting the distal shaft 1 is designated as L1, L1 is preferably 10 mm or more and 100 mm or less. A L1 of less than 10 mm is unfavorable, because there is a concern about the first curved region 10 being expanded and deformed by the core material inserted to the blood removal lumen 8 for prevention of clogging of the blood removal lumen 8 when the distal shaft 1 and the proximal shaft 2 are connected to each other by means of adhesion or heat fusion. Alternatively, a L1 of more than 100 mm is also unfavorable, because it leads to deterioration in operational efficiency, and the torque is less transmitted to the distal shaft 1 in right atrium 25.

When the curvature radius 16 of the circular arc inside the first curved region 10 is designated as $R^1$, $R^1$ is preferably 60 mm or more and 70 mm or less, and, when the circular arc angle 17 is designates as θ1, θ1 is preferably 30° or more and 50° or less. When $R^1$ is less than 60 mm or more than 70 mm, or when θ1 is less than 30° or more than 50°, the most distal region of the blood removal catheter 27 can be introduced less easily through the coronary sinus ostium 26 into coronary sinus. Difficulty in insertion disadvantageously leads to elongation of operation and also increase in the amount of X ray exposure and thus in the load applied to the patient.

Alternatively if the internal curve of the second curved region 11 is elliptic, when the major axis diameter 18 of the ellipse is designated as A1, A1 is preferably 15 mm or more and 21 mm or less, and when the minor axis diameter 19 of the ellipse is designated as B1. B1 is preferably 11 mm or more and 17 mm or less. When A1 is less than 15 mm or more than 21 mm, or when B1 is less than 11 mm or more than 17 mm, the most distal region of the blood removal catheter 27 can be introduced less easily through the coronary sinus ostium 26 into coronary sinus. Difficulty in insertion disadvantageously leads to elongation of operation and also increase in the amount of X ray exposure and thus in the load applied on the patient.

When the length of the second straight region 20 is designated as L2, L2 is preferably 5 mm or more and 15 mm or less. When L2 is less than 5 mm or more than 15 mm, the most distal region of the blood removal catheter can be introduced less easily through the coronary sinus ostium 26 into coronary sinus. Difficulty in insertion disadvantageously leads to elongation of operation and also increase in the amount of X ray exposure and thus in the load applied on the patient.

When the curvature radius 21 of the circular arc inside the third curved region 13 is designated as $R^2$, $R^2$ is preferably 20 mm or more and 30 mm or less, when the circular arc angle 22 is designates as θ2, θ2 is preferably 30° or more and 50° or less. If the $R^2$ is less than 20 mm or more than 30 mm, or when θ2 is less than 30° or more than 50°, the most distal region of the blood removal catheter can be introduced less easily through the coronary sinus ostium 26 into coronary sinus. Difficulty in insertion disadvantageously leads to elongation of operation and also increase in the amount of X ray exposure and thus in the load applied on the patient.

When the length of the third straight region 14 is designated as L3, L3 is preferably 5 mm or more and 20 mm or less. When L3 is less than 5 mm, the most distal region of the blood removal catheter can be introduced less easily through the coronary sinus ostium 26 into coronary sinus. Difficulty in insertion disadvantageously leads to elongation of operation and also increase in the amount of X ray exposure and thus in the load applied on the patient. Unfavorably on the other hand, when L3 is more than 20 mm, there is an increased possibility of the damage of coronary-sinus surrounding wall by the most distal region of the blood removal catheter.

As described above, the distal shaft 1 has three curved regions: a first curved region 10, a second curved region 11, and a third curved region 13. For prevention of the damage of coronary sinus while preservation of the shape of each curved region, the distal shaft 1 is preferably made only of a resin material. The kind of the resin material does not restrict the advantageous effects of the present invention, but the resin material preferably contains an elastomer having a Shore hardness of 25D or more and 75D or less or the blend thereof. Use of an elastomer is effective in making the resin superior both strength and flexibility and giving a blood removal catheter 27 preventing the damage of coronary sinus easily. Single use of an elastomer having a Shore hardness of less than 25D leads to deterioration in strength and is thus unfavorable. Alternatively, single use of an elastomer having a Shore hardness of more than 75D is also unfavorable, because of deterioration in flexibility. The elastomers above may be blended at any rate for obtaining desirable strength and flexibility. Moreover, a softer distal end chip may be formed in the most distal region of the distal shaft 1.

The method of forming the curved shape of the distal shaft 1 does not restrict the advantageous effects of the present invention, and any processing method may be used. A favorable example thereof is a method of forming the curved shape by placing a core material in the blood removal lumen for preservation of the shape and heating the composite in a groove engraved in a curved shape suitable for the desired curve shape.

The blood removal catheter 27 is placed in coronary sinus under monitoring by X-ray radioscopy; the distal tube 1 is preferably visible by X-ray radioscopy for improvement in convenience of manipulation during placement; and for making the tube visible, the resin material preferably contains a X-ray-transmission-prohibiting substance blended.

In addition to the method described above, a marker of X ray-blocking material may be formed on the external or internal surface of the blood removal catheter 27 for making the tube visible, but, for improvement in flexibility of the distal tube 1, the resin material preferably contains a blended X ray-transmission-prohibiting substance.

For example for blending the resin material with the X ray-transmission-prohibiting substance, a method of preparing a distal tube 1 by using a pellet previously obtained by blending the X ray-transmission-prohibiting substance with the resin material for example by biaxial extrusion, or a method of mixing the resin material with the X ray-transmission-prohibiting substance during production of the distal tube 1 may be used, and any method may be used.

The kind of the X ray-transmission-prohibiting substance is not particularly limited, and examples thereof favorably used include compounds containing a metal element such as barium or bismuth, compounds containing a noble metal element such as gold or platinum, and the like, but use of a compound containing a metal element such as barium or bismuth is preferable from the viewpoint of the compatibility with the production process described above. Typical favorable compounds thereof include barium sulfate, bismuth subcarbonate and the like.

The content of the X ray-transmission-prohibiting substance is preferably as high as possible in the range that does not impair the favorable physical properties of the resin significantly and gives favorable molding efficiency. When a favorable compound, barium sulfate or bismuth subcarbonate, is used, the content thereof is preferably 30 wt % or more, more preferably 40 wt % or more.

The blood removal catheter 27 according to the present invention, which is placed through inferior vena cava 24 in coronary sinus, preferably has an effective length of 80 cm or more, more preferably 90 cm or more. For placement of such a blood removal catheter 27 having a relatively long effective length in coronary sinus, the catheter should for example be rotated or pushed forward under application of a certain torque. Therefore, the blood removal catheter 27 according to the present invention should have favorable torque-transmitting efficiency. For that reason, the proximal shaft 2 in the blood removal catheter according to the present invention is preferably a composite tube in combination of a metal material and a resin material. Use of the composite tube assures good torque-transmitting efficiency.

The structure of the composite tube does not restrict the advantageous effects of the present invention, but for improvement of the torque-transmitting efficiency, the metal material is preferably a metal wire having a braid structure, a coil structure, or the structure in combination of the braid and coil structures. The shape, size and material of the metal wire may be selected properly according to the desired physical properties of the composite tube. Thus, metal wires processed into various shapes, such as circular, elliptic, and rectangular shapes, with arbitrary crosssectional dimension are used favorably. A single metal wire or multiple metal wires may be used in forming the braid or coil structure. The number of the metal wires used in a single blood removal catheter is also not particularly limited. Examples of the materials for the metal wire include stainless steels such as SUS304 and SUS316, spring steel, music wire, oil temper wires, Co-Cr alloys, Ni-Ti alloys, and the like.

The resin material for the composite tube is preferably an elastomer having a Shore hardness of 25D or more and 75D or less, and a blend of two or more elastomers may also be used. Use of an elastomer makes the composite tube superior both in strength and flexibility easily. Single use of an elastomer having a Shore hardness of less than 25D is unfavorable, as it results in difficulty in preserving its strength. Alternatively, single use of an elastomer having a Shore hardness of more than 75D is also unfavorable, as it results in deterioration in flexibility. The same resin material may not be used over the entire length of the composite tube, and the physical properties of the composite tube may be altered in the length direction by using two or more resin materials.

The resin material for the distal shaft 1 may be an elastomer identical in Shore hardness with the resin material for the proximal shaft 2 or an elastomer having different Shore hardness, but is preferably a different elastomer, for easier control of the physical properties of the blood removal catheter. A favorable example is a combination of a resin material for the proximal shaft 2 having a Shore hardness of 40D or more and 75D or less and a resin material for the distal shaft 1 having a Shore hardness of 25D or more and 63D or less. Considering the processability and others, the resin materials for the distal shaft 1 and the proximal shaft 2 are preferably polyamide-based elastomers.

The method of producing the composite tube does not restrict the advantageous effects of the present invention. It is produced by forming a tube with a fluorine resin such as a polytetrafluoroethylene (PTFE), a tetrafluoroethylene-perfluoroalkyl vinylether copolymer (PFA), a tetrafluoroethylene-hexafluoropropylene copolymer (FEP), a tetrafluoroethylene-ethylene copolymer (ETFE), polyvinylidene fluoride (PVDF) or polychloro-trifluoroethylene (PCTFE) or a polyamide-based elastomer, winding the metal wires on the external surface thereof in the braid or coil structure, and coating the elastomer above or the elastomer blend additionally on the external surface. The coating methods favorably used include switched extrusion, coating with a heat-shrinkable tube, and the like.

The tube for the distal shaft 1 may be prepared, for example, by extrusion molding, immersion or wire coating.

The catheter is preferably hydrophilized on the external surface, for improvement in the convenience of operation during insertion of the blood removal catheter 27 through inferior vena cava 24 into coronary sinus and for reduction of the concern about the damage of coronary sinus caused by insertion. Hydrophilic coating reduces the abrasion resistance of the surface of the blood removal catheter 27. The kind of the coating material is not limited, and can be selected according to the desired physical properties of the distal shaft 1 and the proximal shaft 2 used and also to the properties of other constituting materials. Examples thereof include hydrophilic polymers such as poly(2-hydroxyethyl methacrylate), polyacrylamide, polyvinylpyrrolidone, and polyethylene glycol. For improvement in adhesiveness to the hydrophilic polymer, the catheter may be surface-treated previously, for example, by plasma treatment or corona discharge treatment or coated with a silane-coupling agent, a urethane-based adhesive or the like. The kind of the coating material or the coating thickness on the blood removal catheter 27 may be altered in the length direction, for gradual increase or decrease in abrasion resistance.

For easier and safer insertion from inferior vena cava 24 into coronary sinus, the blood removal catheter 27 may be guided with a guide wire. In such a case, similarly to when the guide catheter is engaged to coronary artery ostium during PCI, the blood removal catheter 27 with a guidewire placed therein is inserted into the body. An guide wire-insertion lumen (guide wire lumen) may be formed in the blood removal catheter 27, separately with the blood removal lumen 8, but unfavorably, formation of a guide wire lumen without modification of the equivalent diameter of the blood removal lumen 8 (without change in blood removal rate) inevitably results in increase in the external diameter of the blood removal catheter 27. Therefore, the blood removal lumen 8 is preferably used as a guide wire lumen.

If a guide wire is inserted from the hub 3 of the blood removal catheter 27 according to the present invention, particularly if the guide wire distal end has a J-shaped curvature, the guide wire distal end may stick out of the opening 7 in the blood removal catheter 27, making it difficult to guide the catheter with the guide wire. If a guide wire is inserted into a blood removal catheter 27 placed in coronary sinus and the guide wire distal end sticks out of the opening 7, the guide wire distal end should be adjusted to stick out of the distal end of the blood removal catheter 27 by operation of the guide wire by X-ray radioscopy. Such an adjustment under monitoring by X-ray radioscopy demands an extended period of time, increasing the stress to the surgeon and also the load on the patient. Moreover, continued operation without recognition of the guide wire sticking out of the opening by X-ray radioscopy may possibly cause breakage of the blood removal catheter 27 and continued residual of the fragments by collapse of the opening, which is extremely dangerous.

Therefore as shown in Figure 1, the blood removal catheter 27 according to the present invention preferably has a core wire 6 detachably formed in the blood removal lumen 8, and more preferably, the distal end of the core wire 6 is located to the distal side of the opening 7. Use of the core wire 6 thus formed as described above, replacing the guide wire, enables easy and safer insertion of the blood removal catheter 27 into coronary sinus. Moreover, location of the distal end of the core wire 6 to the distal side of the opening 7 distinctively reduces the possibilities of the breakage of the blood removal catheter 27 and the continued residual of the fragments in the body by collapse of the opening 7. Permanent placement of the core wire 6 in the blood removal lumen 8 leads to decrease in the equivalent diameter of the cross section of the blood removal lumen 8 in the circumference direction, prohibiting sufficient blood removal rate. However, use of a detachable core wire 6 in the present invention allows removal of the core wire 6 during blood removal and thus assures sufficient blood removal rate easily.

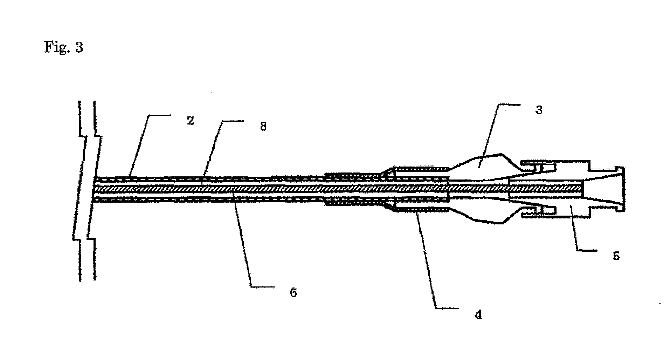

The mechanism of making the core wire 6 detachable is not particularly limited. However, considering the convenience of operation during detachment of the core wire 6, as shown in Figure 3 it is preferable to add a connector 5 to the proximal end of the core wire 6 and make the connector 5 installed detachably from the hub 3. The method of connecting the proximal end of the core wire 6 to the connector 5 does not restrict the advantageous effects of the present invention at all, and the connection may be carried out, for example by adhesion, and the kind of the adhesive for use is also not restricted. The method of connecting the connector 5 to the hub 3 is also not limited, if the connector is detachable, and, in a favorable embodiment, the core wire 6 is made detachable and easily prepared by making the proximal end of the hub 3 in the female luer shape and the connector 5 in the male luer shape.

As described above, if the proximal end of the core wire 6 is connected to the connector 5 and connector 5 to the hub 3 detachably, it becomes possible to flush the internal blood removal lumen 8 through the connector 5. When the blood removal catheter 27 according to the present invention is used, the internal surface of the blood removal lumen 8 should be flushed with a suitable solution such as heparinized physiological saline before insertion into the body. The flushing prevents thrombus formation when the catheter is inserted into the body, particularly into the blood vessel. Normally, the flush is performed by using a syringe. Therefore, the connector 5 in the female luer shape at the proximal end allows flushing while the core wire 6 is inserted and also allows insertion thereof and initiation of therapy soon after flushing.

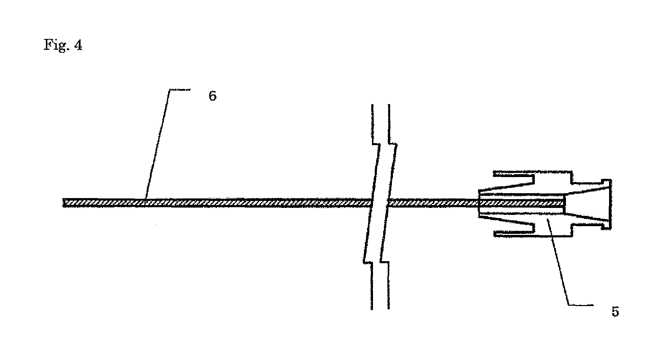

The structure and the shape of the core wire 6 do not restrict the advantageous effects of the present invention at all. A typical example is the straight shape shown in Figure 4. The catheter is preferably wound with metal wires in a spring wire shape for making the distal shaft 1 more flexible. In such a case, the external diameter of the wire constituting the spring wire, the pitch of the springs, and the like are not particularly limited. The pitch of the spring wires may be altered to make the flexibility of the core wire 6 increase toward the distal side in a step wise manner or continuous manner. Alternatively, a core wire may be placed in the spring.

Figure 4 shows a straight shape as a typical example, but a tapered wire may also be used favorably. When such a tapered wire is used, it is possible to control the flexibility of the blood removal catheter 27, by adjusting the taper shape.

The material for the core wire 6 is preferably a metal, for use as a replacement for the guide wire. It is preferably a stainless steel or a Co-Cr alloy, from the viewpoints of corrosion resistance, antithrombogenicity and others. Alternatively, a superelastic alloy may be used, and examples of the favorable superelastic alloys include Ni-Ti alloys, Ni-Ti-Fe alloys, Ni-Ti-Cu alloys, Ni-Ti-Cr alloys, Ni-Ti-V alloys, Ni-Ti-Co alloys, Ni-Ti-Nb alloys, Ni-Ti-Pd alloys, and Ni-Ti-Cu-Cr alloys.

The method of producing the blood removal catheter 27 according to the present invention does not restrict the advantageous effects of the present invention at all. A typical structure of the blood removal catheter 27 is shown in Figure 1, and the distal shaft 1 and the proximal shaft 2, and also the proximal shaft 2 and the hub 3 are connected to each other, and a strain relief 4 for prevention of kink of the blood removal catheter 27 during use is formed in the connection region between the proximal shaft 2 and the hub 3.

The connection method in each connection region is not particularly limited, and may be adhesion by using an adhesive, heat fusion, or the like.

The kind of the adhesive used in the case of adhesion is not limited, and cyanoacrylate-based, urethane-based, silicone-based and epoxy-based adhesives are used favorably. The mechanism of the hardening of the adhesive is also not limited, and adhesive such as two-liquid adhesives, water-hardening adhesives, heat-hardening adhesives, and UV-hardening adhesives are used favorably. Use of an adhesive having, after hardening, a hardness that prohibits discontinuous change in the rigidity of the connection unit in the longitudinal direction, is preferable, and such an adhesive can be selected, for example, according to the kind, size, and rigidity of the material to be bonded. The connection unit may be, for example, heat-treated or abraded for reduction in thickness before and after adhesion, and in the case of a less adhesive material such as polyolefin, the connection unit may be surface-treated for example by plasma treatment.

The material for the hub 3 is not particularly limited, and common injection-moldable resins are used favorably. Examples thereof include polycarbonates, polyamides, polyurethanes, polysulfones, polyarylates, styrene-butadiene copolymers, polyolefin and the like.

The material for the strain relief 4, a part for relaxing the difference in rigidity between the hub 3 and the proximal shaft 2, does not restrict the advantageous effects of the present invention at all. It is preferably a resin material, from the viewpoint of molding processability.

EXAMPLES

Hereinafter, typical Examples and Comparative Examples of the present invention will be described in detail, but it should be understood that the present invention is not limited to the following Examples.

(Example 1)

A composite tube having an external diameter of 2.67 mm, an inner diameter of 2.26 mm, and a length of 900 mm was prepared by using a metal braid of metal wires of 0.10 mm $\times$ 0.03 mm prepared from SUS 304 WPB alloy (16 wires/braid). An internal layer of a polytetrafluoroethylene (Polyflon F-207, DAIKIN INDUSTRIES. LTD) and an external layer of a polyamide elastomer (PEBAX6333 SA01 (Shore hardness: 63D), ATOFINA) was formed by switched extrusion, to give a proximal shaft.

A polyamide elastomer (PEBAX4033 SA01 (Shore hardness: 40D), ATOFINA) and barium sulfate were extruded biaxially into pellets. The barium sulfate content in the pellet was 40 wt %. A tube having an external diameter of 2.60 mm, an internal diameter of 1.85 mm, and a length of 300 mm was prepared by extrusion molding by using the pellets thus prepared. The tube prepared was placed in the grooves (width and depth: 3.1 mm) on a SUS304 alloy plate: one for the first straight region having a length L1 of 10 mm, curvature radius R1 of the circular arc inside the first curved region of 67 mm, and a circular arc angle θ1 of 40°; another for the second curved region having a major axis diameter A1 of 21 mm and a minor axis diameter B1 of 17 mm for the internal ellipse, and the second straight region having a length L2 of 8 mm; and the other for the third curved region having a curvature radius R2 for the internal circular arc of 22 mm, a circular arc angle θ2 of 31° and a third straight region having a length L3 of 20 mm and molded by heating in an oven at 180°C for 45 minutes. A round polytetrafluoroethylene rod (external diameter: 1.80 mm) was placed in the lumen of the tube during the molding for prevention of deformation of the lumen. A circular opening having a diameter of 2.0 mm was formed in the second curved region of the molded tube to the distal side of the vertex by punching, to give a distal shaft.

The distal shaft and the proximal shaft were heat-fused by using hot air at 220°C and a heat-shrinkable tube. During the heat fusion, a round polytetrafluoroethylene rod (external diameter: 1.80 mm) was inserted for prevention of deformation of the lumen. A strain relief of a polyamide elastomer (PEBAX5533SA01, ATOFINA) and a hub of polycarbonate (Makloron2658, Bayer) were connected each other by using a two-liquid urethane-based adhesive (UR0531, H.B. Fuller), to give a blood removal catheter.

(Example 2)

A blood removal catheter was prepared in a similar manner to Example 1, except that L1 was changed to 50 mm, R1 to 70 mm, θ1 to 40°, A1 to 15 mm, B1 to 11 mm, L2 to 15 mm, R2 to 25 mm, θ2 to 50°, and L3 to 10 mm, and a circular opening having a diameter of 1.8 mm was formed.

(Example 3)

A blood removal catheter was prepared in a similar manner to Example 1, except that the external diameter of the composite tube was changed to 3.33 mm, the internal diameter to 2.72 mm, the tube external diameter to 3.00 mm, and the internal diameter to 2.25 mm, L1 was changed to 100 mm, R1 to 60 mm, θ1 to 50°, A1 to 15 mm, B1 to 11 mm, L2 to 5 mm, R2 to 20 mm, θ2 to 30°, and L3 to 5 mm, and a round polytetrafluoroethylene rod (external diameter: 2.20 mm) was inserted into the tube lumen during molding.

(Example 4)

A blood removal catheter was prepared in a similar manner to Example 3, except that L1 was changed to 40 mm, R1 to 65 mm, θ1 to 30°, A1 to 21 mm, B1 to 17 mm, L2 to 10 mm, R2 to 30 mm, θ2 to 35°, and L3 to 15 mm.

(Example 5)

A blood removal catheter was prepared in a similar manner to Example 1, except that a circular opening having a diameter of 1.8 mm was formed in the third curved region.

(Comparative Example 1)

A blood removal catheter was prepared in a similar manner to Example 1, except that L1 was changed to 30 mm, R1 to 67 mm, θ1 to 40°, A1 to 21 mm, B1 to 17 mm, L2 to 8 mm, R2 to 30 mm, θ2 to 60°, and L3 to 25 mm.

(Comparative Example 2)

A blood removal catheter was prepared in a similar manner to Example 1, except that L1 was changed to 10 mm, R1 to 67 mm, θ1 to 40°, A1 to 35 mm, B1 to 25 mm, L2 to 20 mm, R2 to 22 mm, θ2 to 60°, and L3 to 0 mm.

(Evaluation)

A 7Fr sheath introducer was inserted into the left femoral artery of a LWD pig (body weight: 63.6 kg) under inhalation anesthesia. A 7Fr guiding catheter was placed through the sheath introducer in the left main trunk. A 5Fr guiding catheter was inserted in the 7Fr guiding catheter and the distal end was placed in the origin of circumflex branch of left coronary blood vessel.
Separately, a 10Fr sheath introducer was inserted into the right femoral vein. Each of the blood removal catheters in Examples and Comparative Examples was introduced through the sheath introducer and placed in coronary sinus under observation by X-ray radioscopy.
After placement, the blood was removed by using an extracorporeal circulation device DX21 (manufactured by Kaneka Corporation) while an extension tube having a length of 1,000 mm was connected to the hub of the blood removal catheter, and the blood removal pressure and the blood removal rate were measured. The blood removed was fed back into left femoral vein through a 16G detention needle inserted thereto.
After blood removal for 10 minutes, a contrast medium (Iopamiron 370, Nihon Schering K.K) was administered at a flow rate of 2.5 mL/min continuously for 2 minutes through a guiding catheter located at the origin of the circumflex branch of left coronary blood vessel. Simultaneously with administration, the blood was collected at a rate of 1.0 mL/min by using a tube pump connected to the three-way valve formed in the extension tube. Sampling was continued for 14 minutes. The blood collected in 1-mL vacuum blood sample tubes containing EDTA-2K was made anticoagulant and centrifuged for separation of the blood plasma. The concentration of the contrast medium in the blood plasma was determined, and the recovery rate of the contrast medium administered was calculated by the following Formula.

Formula 1

Recovery rate [%]=[{(Contrast medium concentration in sample blood × Blood sampled)× (Blood removal rate/sampling rate)}/Contrast medium administered] $\times$ 100

(Results)

The catheters of Examples 1 to 4 according to the present invention could be placed easily in coronary sinus. In addition, the catheter of Example 5, which is identical with that of Example 1 in the shape of the distal shaft but differs only in the position of the opening, could be placed in coronary sinus similarly to those in Examples 1 to 4. However, the catheters of Comparative Examples 1 and 2 could not be placed in coronary sinus.

Blood was removed by using each of the catheters of Examples 1 to 5 that could be placed in coronary sinus. Blood removal and recirculation at blood removal rate of 140 mL/min were possible with the catheters of Examples 1 to 4. The blood removal pressure with the catheter of Example 1 was -76 mmHg; that of Example 2, -85 mmHg; that of Example 3, -68 mmHg; and that of Example 4, -63 mmHg; and thus, the blood was removed safely in all cases, at a high blood removal pressure of higher than -100 mmHg. On the other hand, with the catheter of Example 5, the blood removal pressure declines rapidly, prohibiting blood removal, at a blood removal rate of 80 mL/min or more. Therefore, the blood removal was continued at a rate of 80 mL/min, but the blood removal pressure remained low at -80 mmHg, even though the blood removal rate was reduced.

Separately, a contrast medium was administered from the circumflex branch of left coronary blood vessel and the recovery rate of the contrast medium was monitored. As a result, the recovery rate in Example 1 was 86%; in Example 2, 77%; in Example 3, 59%; and in Example 4, 80%; indicating that use of a blood removal catheter according to the present invention was extremely effective in removing the administered contrast medium from the body. The recovery rate by the catheter of Example 5 was 40%.

## Claims

1.   A blood removal catheter for removing blood in coronary sinus out of the body that is placed in the coronary sinus through inferior vena cava, the blood removal catheter comprising:

a straight proximal shaft;
a curved distal shaft located to the distal side of the proximal shaft and connected to the straight proximal shaft;
a hub connected to the proximal side of the proximal shaft; and
a blood removal lumen extending in the blood removal catheter from the proximal end to distal end, wherein the distal shaft comprises:

a first straight region constituting the connection part to the proximal shaft;
a first curved region connected to the distal side of the first straight region;
a second curved region connected to the distal side of the first curved region and bending in the direction opposite to that of the first curved region;
a second straight region connected to the distal side of the second curved region;
a third curved region connected to the distal side of the second straight region and bending in the direction opposite to that of the second curved region; and
a third straight region connected to the distal side of the third curved region and constituting the most distal region of the blood removal catheter, wherein:

the first and third curved regions form part of circular arcs;
the second curved region forms a part of an ellipse having a minor axis and a major axis;
the part of the ellipse has a starting point at one end of the minor axis and a terminal point at the other end of the minor axis;
at least one opening having an equivalent diameter of 1.8 mm or more is formed at a position to the distal side from the vertex in the second curved region; and
the equivalent diameter of the cross section of the blood removal lumen in the circumference direction is 1.8 mm or more over the entire length of the blood removal lumen.

2.   The blood removal catheter according to Claim 1, wherein the length L1 of the first straight region is 10 mm or more and 100 mm or less.

3.   The blood removal catheter according to Claim 1 or 2,, wherein the first curved region is circular arc inside; the curvature radius R1 of the circular arc is 60 mm or more and 70 mm or less; and the circular arc angle θ1 is 30° or more and 50° or less.

4.    The blood removal catheter according to any one of Claims 1 to 3, wherein the second curved region is partially elliptic inside; the ellipse has a major axis diameter A1 of 15 mm or more and 21 mm or less and a minor axis diameter B1 of 11 mm or more and 17 mm or less; and part of the ellipse extends from a starting point of one end of the minor axis to a terminal point at the other end of the minor axis.

5.    The blood removal catheter according to any one of Claims 1 to 4, wherein the length L2 of the second straight region is 5 mm or more and 15 mm or less.

6.    The blood removal catheter according to any one of Claims 1 to 5, wherein the third curved region is circular arc inside; the curvature radius R2 of the circular arc is 20 mm or more and 30 mm or less; and the circular arc angle θ2 is 30° or more and 50° or less.

7.    The blood removal catheter according to any one of Claims 1 to 6, wherein the length L3 of the third straight region is 5 mm or more and 20 mm or less.

8.    The blood removal catheter according to any one of Claims 1 to 7, wherein the distal shaft is made only of a resin material.

9.    The blood removal catheter according to Claim 8, wherein the resin contains an X ray-transmission-prohibiting substance as it is blended.

10.   The blood removal catheter according to any one of Claims 1 to 9, wherein the proximal shaft is a composite tube in combination of a metal material and a resin material.

11.   The blood removal catheter according to Claim 10, wherein the metal material is a metal wire and the metal wire has a braid structure.

12.   The blood removal catheter according to Claim 10, wherein the metal material is a metal wire and the metal wire has a coil structure.

13.   The blood removal catheter according to any one of Claims 1 to 12, further comprising a core wire detachably placed in the blood removal lumen.

14.   The blood removal catheter according to Claim 13, wherein the core wire has a connector at the proximal end and the connector is placed detachably from the hub.

15.   The blood removal catheter according to Claim 13 or 14, wherein the distal end of the core wire is located to the distal side of the opening.

Fig. 1

Fig. 2

Fig. 3

Fig. 4

Fig. 5

25

26

24

27

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7303701 A

**Non-patent literature cited in the description**

- *Coronary Intervention,* 2003, vol. 2 (4), 78-83